# EUROPEAN PATENT SPECIFICATION

(11) **EP 1 670 519 B1**
(45) Date of publication and mention of the grant of the patent: **13.07.2016**
(21) Application number: 04788738.5
(22) Date of filing: 13.09.2004
(51) Int. Cl.: A61K 51/00, C07H 19/06, C07H 1/00

(54) **RADIOLABELED THYMIDINE SOLID-PHASE EXTRACTION PURIFICATION METHOD**
FESTPHASENEXTRAKTIONSREINIGUNGSVERFAHREN FÜR RADIOAKTIV MARKIERTES THYMIDIN
PROCEDE DE PURIFICATION PAR EXTRACTION EN PHASE SOLIDE DE THYMIDINE RADIOMARQUEE

(30) Priority: 11.09.2003 US 501922 P
(43) Date of publication of application: 21.06.2006
(73) Proprietor: MAYO FOUNDATION FOR MEDICAL EDUCATION AND RESEARCH, Rochester, MN 55905 (US)
(72) Inventor: TRUMP, David, P., Byron, MN 55920 (US)
(74) Representative: Gervasi, Gemma
(86) International application number: PCT/US2004/029988
(87) International publication number: WO 2005/025519

(56) References cited:
- MACHULLA H J ET AL: "SIMPLIFIEDLABELING APPROACH FOR SYNTHESIZING 3'-DEOXY-3'- not 15F 3/4 FLUOROTHYMIDINE( not 18F 3/4 FLT)" JOURNAL OF RADIOANALYTICAL AND NUCLEAR CHEMISTRY, ARTICLES, ELSEVIER SEQUOIA S.A., LAUSANNE, CH, vol. 243, no. 3, 1 January 2000 (2000-01-01), pages 843-846, XP008003386
- BLOCHER A ET AL: "Synthesis and labeling of 5'-O-(4,4'-dimethoxytrityl)-2,3'-anhydroth y midine for Ä18FÜFLT preparation" JOURNAL OF RADIOANALYTICAL AND NUCLEAR CHEMISTRY, ARTICLES, ELSEVIER SEQUOIA S.A., LAUSANNE, CH, vol. 251, no. 1, 1 January 2002 (2002-01-01), pages 55-58, XP002989061
- GRIERSON J R ET AL: "Radiosynthesis of 3'-deoxy-3'-[<18>F]fluorothymidine: [<18>F]FLT for imaging of cellular proliferation in vivo" NUCLEAR MEDICINE AND BIOLOGY, ELSEVIER, NY, US, vol. 27, no. 2, 1 February 2000 (2000-02-01), pages 143-156, XP004196582 ISSN: 0969-8051
- MACHULLA H.J. ET AL: 'Simplified labeling approach for synthesizing 3'-deoxy-3'-[18F]fluorothymidine ([18F]FLT)' JOURNAL OF RADIOANALYTICAL AND NUCLEAR CHEMISTRY vol. 243, no. 3, 2000, pages 843 - 846, XP008003386
- BLOCHER A. ET AL: ' Synthesis and labeling of 5'-O-(4,4'-dimethoxytrityl)-2,3'-anhydrothy midine for [18F]FLT preparation' JOURNAL OF RADIOANALYTICAL AND NUCLEAR CHEMISTRY vol. 251, no. 1, 2002, pages 55 - 58, XP002989061
- J.R. GRIERSON ET AL.: "Development of a radiosynthesis for 3'-[F-18]fluoro-3'-deoxynucleosides", J. LABELLED COMPOUNDS RADIOPHARM., vol. 40, 1997, pages 60-62,

## Description

### BACKGROUND OF THE INVENTION

### 1. Field of the Invention

The invention relates to a method and an apparatus for preparing 3'-deoxy-3' [¹⁸F]Fluorothymidine.

### 2. Description of the Related Art

Radiolabeled compounds are used as diagnostic imaging agents in positron emission tomography (PET). For example, 2- [¹⁸F] fluoro-2-deoxy-D- glucose (FDG) is one tracer used in nuclear medical imaging. This molecule, labeled with the radionuclide ¹⁸F, behaves in a way similar to glucose in the first step of its destabilization in the human body and allows a user to map and quantify this fundamental mechanism. It is indicated for diagnosis of numerous diseases. FDG and its preparation are described in U. S. Patent Nos. 4,617, 386, 4,794, 178,5, 169,942, 5,264, 570,5, 436,325, 5,759, 513,5, 808,020, 5,932, 178, and 6,172, 207.

Another radiolabeled compound, radiolabeled thymidine, is currently being explored as a diagnostic imaging agent for positron emission tomography in the area of oncology. There is much excitement being generated about its possibilities. However, radiolabeled thymidine is difficult to produce so its use has been limited. The current method used to purify 3'-deoxy-3'-[¹⁸F] Fluorothymidine employs high-pressure liquid chromatography (HPLC). This technique requires a column, high pressure pumps, and an ultraviolet detector which is a relatively complex system. Such systems can range in price from a few thousand dollars up to $30,000 and they require a 30 minute set up procedure and about 10 minutes for each synthesis use. Because of their complexity, prior systems are also less reliable, particularly in a clinical setting.

Thus, the current method used to purify 3'-deoxy-3'- [¹⁸F] Fluorothymidine presents many problems. For instance, the long purification time for 3'-deoxy-3'- [¹⁸F] Fluorothymidine is undesirable given the 110 minute half life for the ¹⁸F fluoride ion; and the automated 3'-deoxy-3'-[¹⁸F] Fluorothymidine purification equipment has a high cost.

J.R. Grierson et al. describe in their publication "Development of a radiosynthesis for 3'-[F-18]fluoro-3'deoxynucleosides" (J. Labelled Compounds radiopharm., vol 40, 1997, pages 60 - 62) a method for the synthesis of 3'-deoxy-3'-[¹⁸F]fluorothymidine which is isolated by using a Sep-Pak Plus C-18 column, which is eluted with acetonitril, and subsequently by using HPLC.

Therefore, there is a need for a method and an apparatus for preparing purified 3'-deoxy-3'- [¹⁸F] Fluorothymidine at lower cost with decreased purification times.

### SUMMARY OF THE INVENTION

The present invention provides a method and apparatus for preparing 3'-deoxy-3'-[¹⁸F]Fluorothymidine according to claims 1 and 13, respectively.. This method replaces more complicated, costly, and time- consuming purification methods like high-pressure liquid chromatography purification. This method has also been incorporated into an automated synthesis unit to purify radiolabeled thymidine without human intervention after production has started.

The solid-phase trapping method of the present invention reduces purification time from approximately 10 minutes to 2 minutes or less. The cost of the previous equipment needed for current purification is several thousand dollars depending on the exact set-up. The cost for this invention is only a few dollars per synthesis. The simple nature of this invention (i. e., lack of electronic parts, detectors, complex valves) improves reliability, which is very important in the clinical setting.

In one aspect, the invention for preparing 3'-deoxy-3'- [¹⁸F]Fluorothymidine according to claims 1. In the method, a fluorothymidine precursor, a reaction solvent for the fluorothymidine precursor, a source of [¹⁸F] ions, and dilution water are combined to thereby form a reaction mixture including a radiolabeled thymidine product, [¹⁸F] ions, and residual reaction impurities. The term"reaction mixture"does not require that all components of the reaction mixture participate in a chemical reaction.) The reaction mixture is passed through a solid phase extraction cartridge as a first extraction step. The solid phase extraction cartridge includes a solid sorbent to adsorb the radiolabeled 3'-deoxy-3'-[¹⁸F]Fluorothymidine on the sorbent, and at least a portion of the residual reaction impurities pass through solid phase extraction cartridge without adsorbing on the sorbent. The solid phase extraction cartridge sorbent is preferably a reversed phase C-18 sorbent. Any remaining impurities on the sorbent are washed off with wash water, and the 3'-deoxy-3'-[¹⁸F]Fluorothymidine is eluted off the sorbent using an alkanol solvent. The eluted radiolabeled 3'-deoxy-3'-[¹⁸F]Fluorothymidine is dried and reconstituted in water before any further extraction steps. According to the present invention, the method further comprises passing the reconstituted radiolabeled 3'-deoxy-3'-[¹⁸F]Fluorothymidine through a solid phase extraction cartridge.

The solid phase extraction cartridge may be the only reversed phase solid phase extraction cartridge used in the method, and according to the present invention, high pressure liquid chromatography is not used in the method. Preferably, the alkanol solvent is ethanol, and the reaction solvent is an aprotic solvent such as dimethyl sulfoxide. The dilution water that is combined with the fluorothymidine precursor, the reaction solvent for the fluorothymidine precursor, and the source of [¹⁸F] ions is in an amount in excess of the reaction solvent, and the wash water used to wash off any remaining impurities on the sorbent is preferably not delivered by way of the reaction vessel.

The reaction mixture may be passed through a filter before passing the reaction mixture through the solid phase extraction cartridge. The filter removes precipitates from the reaction mixture. Also, bypassing the filter after passing the reaction mixture through the filter for a time period is possible. Preferably, the steps of passing the reaction mixture through the solid phase extraction cartridge, washing off the remaining impurities on the sorbent, and eluting the radiolabeled thymidine product off the sorbent take two minutes or less.

The reconstituted radiolabeled thymidine product is passed through a solid phase extraction cartridge to further remove impurities and thereafter filtered to further remove impurities.

In another aspect, the invention provides an apparatus for preparing a radiolabeled 3'-deoxy-3'-[¹⁸F]Fluorothymidine. The apparatus includes a reaction vessel for containing a fluorothymidine precursor, a reaction solvent for the fluorothymidine precursor, a source of [¹⁸F] ions, and dilution water to thereby form a reaction mixture including a radiolabeled 3'-deoxy-3'-[¹⁸F]Fluorothymidine, [¹⁸F] ions, and residual reaction impurities. A solid phase extraction cartridge is in fluid communication with the reaction vessel. The solid phase extraction cartridge includes a solid sorbent to adsorb the radiolabeled thymidine product on the sorbent. At least a portion of the residual reaction impurities pass through solid phase extraction cartridge without adsorbing on the sorbent. The apparatus includes a source of wash water in fluid communication with the solid phase extraction cartridge for providing wash water to wash off any remaining impurities on the sorbent with water, and a source of an alkanol solvent in fluid communication with the solid phase extraction cartridge for providing the alkanol solvent to elute the radiolabeled thymidine product off the sorbent using the alkanol solvent. The apparatus does not include a high pressure liquid chromatography unit.

Preferably, the wash water is not delivered by way of the reaction vessel.

The apparatus may include a filter in fluid communication with the reaction vessel and the solid phase extraction cartridge such that the reaction mixture passes through the filter before the solid phase extraction cartridge. The filter removes precipitates from the reaction mixture. There may be provided a bypass conduit in fluid communication with the reaction vessel and the solid phase extraction cartridge for allowing the reaction mixture to bypass the filter.

The apparatus may further include a controller and a controllable valve in electrical communication with the controller. The controllable valve is located between the reaction vessel and the bypass conduit. There is provided a pressure sensor in electrical communication with the controller. The pressure sensor provides a signal to the controller when the filter is clogged such that the controller adjusts the controllable valve to allow the reaction mixture to bypass the filter.

for the method for preparing 3'-deoxy-3'-[¹⁸F]Fluorothymidine may comprise preparing a radiolabeled 3'-deoxy-3'-[¹⁸F]Fluorothymidine on a radiolabeled product synthesizer that has a reaction vessel and a high pressure liquid chromatography unit in a product flow path. In this method, the high pressure liquid chromatography unit is removed from the product flow path, and a fluorothymidine precursor, a reaction solvent for the fluorothymidine precursor, a source of [¹⁸F] ions, and dilution water are combined in the reaction vessel to thereby form a reaction mixture including a radiolabeled thymidine product, [¹⁸F] ions, and residual reaction impurities. The reaction mixture is passed through a solid phase extraction cartridge, which includes a solid sorbent to adsorb the radiolabeled thymidine product on the sorbent. In one form, the solid phase extraction cartridge sorbent is a C-18 sorbent. At least a portion of the residual reaction impurities pass through solid phase extraction cartridge without adsorbing on the sorbent. Any remaining impurities on the sorbent are washed off with wash water, and the radiolabeled thymidine product is eluted off the sorbent using an alkanol solvent such as ethanol.

The dilution water that is combined with the fluorothymidine precursor, the reaction solvent for the fluorothymidine precursor, and the source of [¹⁸F] ions is in an amount in excess of the reaction solvent (e. g., dimethyl sulfoxide).

Preferably, the fluorothymidine precursor, the reaction solvent for the fluorothymidine precursor, the source of [¹⁸F] ions, and water are combined in a reaction vessel, and the wash water used to wash off any remaining impurities on the sorbent is not delivered by way of the reaction vessel.

The reaction mixture may be passed through a filter before passing the reaction mixture through the solid phase extraction cartridge so that the filter removes precipitates from the reaction mixture. The filter may be bypassed after passing the reaction mixture through the filter for a time period. Preferably, the steps of passing the reaction mixture through a solid phase extraction cartridge, washing off the remaining impurities on the sorbent, and eluting the radiolabeled thymidine product off the sorbent take two minutes or less.

These and other features, aspects, and advantages of the present invention will become better understood upon consideration of the following detailed description, drawings, and appended claims.

### BRIEF DESCRIPTION OF THE DRAWINGS

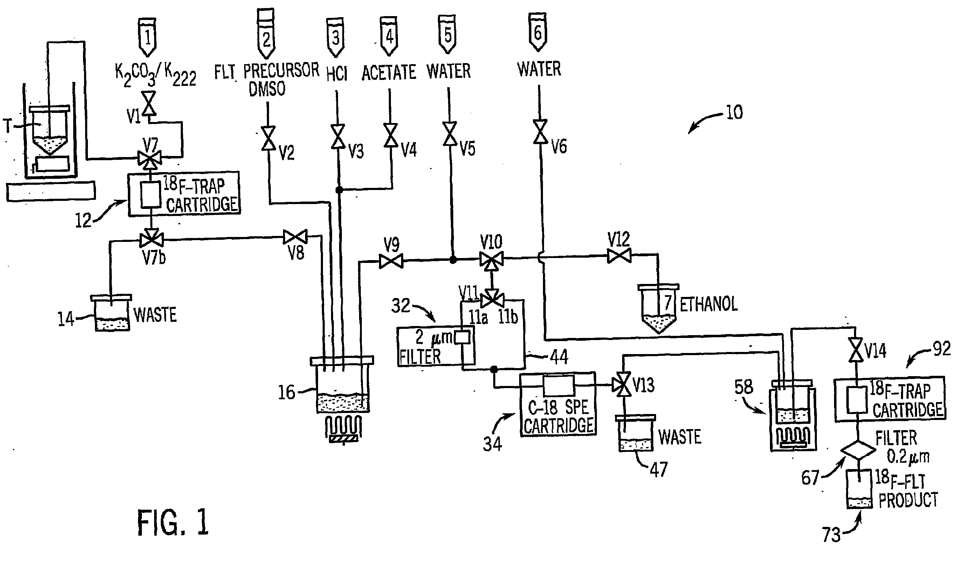

Fig. 1 is a schematic diagram of a synthesizer for practicing the present invention.

### DETAILED DESCRIPTION OF THE INVENTION [

Referring to Figure 1, there is shown a synthesizer 10 according to the present invention for preparing 3'-deoxy-3'- [¹⁸F] Fluorothymidine. A suitable combined in the reaction vessel to thereby form a reaction mixture including a radiolabeled thymidine product, [¹⁸F] ions, and residual reaction impurities. The reaction mixture is passed through a solid phase extraction cartridge, which includes a solid sorbent to adsorb the radiolabeled thymidine product on the sorbent. In one form, the solid phase extraction cartridge sorbent is a C-18 sorbent. At least a portion of the residual reaction impurities pass through solid phase extraction cartridge without adsorbing on the sorbent. Any remaining impurities on the sorbent are washed off with wash water, and the radiolabeled thymidine product is eluted off the sorbent using an alkanol solvent such as ethanol.

The dilution water that is combined with the fluorothymidine precursor, the reaction solvent for the fluorothymidine precursor, and the source of [¹⁸F] ions is in an amount in excess of the reaction solvent (e.g., dimethyl sulfoxide). Preferably, the fluorothymidine precursor, the reaction solvent for the fluorothymidine precursor, the source of [¹⁸F] ions, and water are combined in a reaction vessel, and the wash water used to wash off any remaining impurities on the sorbent is not delivered by way of the reaction vessel.

The reaction mixture may be passed through a filter before passing the reaction mixture through the solid phase extraction cartridge so that the filter removes precipitates from the reaction mixture. The filter may be bypassed after passing the reaction mixture through the filter for a time period. Preferably, the steps of passing the reaction mixture through a solid phase extraction cartridge, washing off the remaining impurities on the sorbent, and eluting the radiolabeled thymidine product off the sorbent take two minutes or less.

These and other features, aspects, and advantages of the present invention will become better understood upon consideration of the following detailed description, drawings, and appended claims.

### BRIEF DESCRIPTION OF THE DRAWINGS

Fig. 1 is a schematic diagram of a synthesizer for practicing the present invention.

### DETAILED DESCRIPTION OF THE INVENTION

Referring to Figure 1, there is shown a synthesizer 10 according to the present invention for preparing 3'-deoxy-3'-[¹⁸F]Fluorothymidine. A suitable general reaction scheme for preparing 3'-deoxy-3'-[¹⁸F]Fluorothymidine is described by Machulla et al. at "Simplified labeling approach for synthesizing 3'-deoxy-3'-[18F]fluorothymidine ([18F]FLT)", Journal of Radioanalytical and Nuclear Chemistry, 24: 843-846, 2000. In synthesizer 10, vessels are provided for containing various reactants used in preparing 3'-deoxy-3'-[¹⁸F]Fluorothymidine. Specifically, vessel T is provided for containing O-18(¹⁸O) water including [¹⁸F] fluoride ions (hereinafter the "target water"); vessel 1 is provided for containing potassium carbonate (K₂CO₃) and Kryptofix™ 222 (k₂₂₂); vessel 2 is provided for containing a fluorothymidine (FLT) precursor and dimethylsulfoxide (DMSO) solvent; vessel 3 is provided for containing hydrochloric acid (HCl); vessel 4 is provided for containing an aqueous solution of sodium acetate; vessels 5 and 6 are provided for containing water; and vessel 7 is provided for containing ethanol. Controllable valves V1 to V14 are provided in various flow conduits for controlling the movement of fluid materials in the synthesizer 10.

In operation, the target water including O-18(¹⁸O) water and [¹⁸F] fluoride ions (which may be synthesized using known techniques such as those described in U.S. Patent Nos. 6,567,492 and 6,190,637) is delivered from vessel T through valve V7 and passed through a [¹⁸F] trap cartridge 12 such as an anion-exchange resin. The [¹⁸F] trap cartridge 12 traps the [¹⁸F] ions in the target water and the waste target water passes through valve V7b into waste container 14.

An aqueous potassium carbonate solution and an acetonitrile (CH₃CN) solution of Kryptofix™ 222 are then delivered from vessel 1, and are passed through the [¹⁸F] trap cartridge 12 to extract the [¹⁸F] ions trapped by means of the [¹⁸F] trap cartridge 12. The Kryptofix™ 222 is an activating agent that activates the [¹⁸F] by making it more reactive. The thus extracted [¹⁸F] fluoride ion is sent via valve V8 to a reaction vessel 16. Subsequently, the reaction vessel 16 including the [¹⁸F] fluoride ion, the potassium carbonate and the acetonitrile solution of Kryptofix™ 222 is heated under reduced pressure to eliminate moisture in the reaction vessel 16.

After the mixture in the reaction vessel 16 is sufficiently dried, the fluorothymidine (FLT) precursor and dimethylsulfoxide (DMSO) solvent are delivered from vessel 2 through valve V2 into the reaction vessel 16, which is then heated. The fluorothymidine (FLT) precursor used may be a dimethoxytriphenylmethyl protected anhydrothymidine known as 5'-O-(4,4' dimethoxytriphenylmethyl)-2-3'-anhydrothymidine. Other protected anhydrothymidines may also be suitable. In one example embodiment, 10 milligrams of 5'-O-(4,4' dimethoxytriphenylmethyl)-2-3'-anhydrothymidine dissolved in 1 milliliter of DMSO are delivered from vessel 2 through valve V2 into the reaction vessel 16.

Hydrochloric acid (HCl) is then delivered through valve V3 to the reaction vessel 16 to deprotect the 5'-O-(4,4' dimethoxytriphenylmethyl)-2-3'-anhydrothymidine. An aqueous solution of sodium acetate is then delivered through valve V4 to the reaction vessel 16 to adjust the pH of the reaction mixture towards neutral. Preferably, the aqueous solution of sodium acetate includes dilution water in an amount such that the volume ratio of dilution water to DMSO in the reaction vessel is at least 7 to 1 and preferably 9 to 1. After the reaction, [¹⁸F] fluoride ions, Kryptofix™ 222, potassium carbonate, acetonitrile, HCl, sodium acetate, water and the radiolabeled 3'-deoxy-3'-[¹⁸F]Fluorothymidine may be present in the reaction vessel 16. Some of these (e.g., acetonitrile) may not remain in the reaction vessel 16.

The reaction mixture of any of the [¹⁸F] fluoride ions, Kryptofix™ 222, potassium carbonate, acetonitrile, HCl, sodium acetate, dilution water and the radiolabeled 3'-deoxy-3'-[¹⁸F]Fluorothymidine are then delivered from the reaction vessel 16 through valves V9 and V10 and valve V11 side 11 a and through frit 32 (pore size = 2 microns) to a solid phase extraction cartridge 34. Suitable solid phase extraction cartridges include those sold commercially as the Waters Sep-Pak® Plus C-18 solid phase extraction cartridge or the Waters Sep-Pak® C-18 Light. These types of cartridges are also described in U.S. Patent Nos. 4,211,658 and 4,250,035, and their use is described in "Solid Phase Extraction Applications Guide and Bibliography" Sixth Edition 1995 published by Waters. The C-18 reversed-phase solid sorbent is a strongly hydrophobic silica-based bonded phase with a monofunctional bonding chemistry and a -Si(CH₃)₂C₁₈H₃₇ surface functionality . The nominal pore size is 125 Angstroms and the particle size range is typically 55-105 micrometers.

The use of frit 32 serves to filter the reaction mixture prior to loading on the solid phase extraction cartridge 34. It has been discovered that water may precipitate reaction mixture components, which would clog solid phase extraction cartridge 34, rendering it ineffective. Various counter measures are taken to avoid precipitate clogging of the solid phase extraction cartridge 34. For example, the frit (pore size = 2 micron) blocks the appropriate amount of solid reaction mixture components to prevent the solid phase extraction cartridge from clogging. Also, a frit bypass line 44 is provided if there is a need to have the option to bypass the frit 32 because it gets partially blocked, slowing the filtration. The cartridge 34 can still work normally when the bypass line 44 is used, provided that most of the reaction mixture has been filtered. The limits of solid material deposition on the cartridge 34 have been explored and with the synthesizer 10 the process described above is reliable.

With respect to solid phase extraction cartridge selection, the Waters Sep-Pak® C-18 solid phase extraction cartridge is suitable because C-18 tends to retain lipophilic compounds when they are loaded in hydrophilic mixtures. The DMSO is miscible with water and needs to be greatly diluted with water for the concept to work best. The concept was explored and dilution can be optimized to allow retention of ¹⁸F-FLT (3'-deoxy-3'-[¹⁸F]Fluorothymidine). The pressure must also be controlled, to keep the flow slow enough to allow the ¹⁸F-FLT to interact with the solid-phase material in the solid phase extraction cartridge 34.

After delivering the reaction mixture to the solid phase extraction cartridge 34, ¹⁸F-FLT and a small amount of impurities are retained on the solid-phase material in the solid phase extraction cartridge 34. The ¹⁸[F] fluoride ions, Kryptofix™ 222, potassium carbonate, acetonitrile, HCl, sodium acetate, and water move through the cartridge 34 and are sent to waste vessel 47 by way of valve V13.

Water is then delivered through valves V5, V10 and V11 and through the solid phase extraction cartridge 34 to wash the remaining impurities off the solid-phase sorbent material in the solid phase extraction cartridge 34. Washing the cartridge 34 with water works well to remove the hydrophilic ¹⁸F fluoride ions, and one key is using an adequate supply of water to ensure complete fluoride removal. In one embodiment, the impurities are washed away from the cartridge 34 using about 15.5 milliliters of water. The remaining impurities and water are sent to waste vessel 47 by way of valve V13. Purified ¹⁸F-FLT remains on the solid-phase material in the solid phase extraction cartridge 34.

Ethanol is then delivered through valves V12, V10 and V11 through the solid phase extraction cartridge 34 to elute the purified ¹⁸F-FLT off the solid-phase material in the solid phase extraction cartridge 34. The ethanol and purified ¹⁸F-FLT mixture is then delivered to vessel 58 by way of valve V13. In one embodiment, the purified product is eluted from the cartridge 34 using 1 milliliter of ethanol. Ethanol is a suitable eluent, and it has a low toxicity in humans. It also evaporates at low temperatures (to avoid decomposition of the product), and was found to adequately elute the product from the cartridge 34. The ethanol and purified ¹⁸F-FLT mixture in the vessel 58 are heated to remove the ethanol. After evaporation of the ethanol, the purified ¹⁸F-FLT is dissolved in water delivered to vessel 58 by way of valve V6. Water (not saline) is used because the addition of ions to the mixture would interfere with removal of residual ¹⁸F fluoride ions at a later step.

Water may not dissolve the ¹⁸F-FLT product adequately. Therefore, the water and ¹⁸F-FLT mixture in the vessel 58 may be delivered through valve V14 after reconstitution to a second [¹⁸F] fluoride trap 92 similar to the [¹⁸F] trap cartridge 12 and a filter 67 (0.2 micron pore size) for filtering and delivery to vessel 73 prior to any further fluoride removal and sterilization. This prevents clogging of cartridges and sterilization filters. Usually, HPLC would be used to remove ¹⁸F fluoride ions, but the second [¹⁸F] fluoride trap 92 was used as an alternative and was found to effectively reduce fluoride in the product to make the product suitable for use.

The entire synthesizer 10 may be operated by a controller using suitable software for control of, among other things, valves V1 to V14. This provides many advantages including, for example, software accommodation of the frit bypass line 44. A pause may be built-into the frit filtering step. The valves are positioned such that a pressure drop can be observed by a user to ensure that the pressure is dropping as expected while material is passing through the frit 32. If pressure does not drop during observation, it indicates that the frit 32 is clogged, and frit bypass line 44 should be used.

Various reaction mixtures are described in the literature for labeling fluorothymidine. The example mixture described above for the invention incorporates dimethylsulfoxide (DMSO) as a solvent. DMSO was desired as a solvent in this purification process because it dissolves the precursor molecule better than the alternative solvents. The need for optimal dissolution (for increased labeling efficiency) was important due to the fact that solid phase extraction methods can be inefficient at purification. The negative aspects of using DMSO were taken into account (high boiling point, tendency to leave residual) and determined to be tolerable with taking the appropriate counter measures such as (i) DMSO dilution with large excess of water (at least 7 to 1 water to DMSO) to prevent DMSO from eluting ¹⁸F-FLT off the cartridge 34 while loading the diluted reaction mixture. (the large excess of water also starts to minimize effect of residual DMSO due to decreasing concentration), and (ii) water wash phase delivery of water through an independent path from the reaction vessel to prevent residual DMSO in the reaction vessel 16 from contaminating the lines and solid phase extraction cartridge 34.

## Claims

1. A method for preparing 3'-deoxy-3'-[¹⁸F]Fluorothymidine, the method comprising:
combining a fluorothymidine precursor, a reaction solvent for the fluorothymidine precursor, a source of [¹⁸F] ions, and dilution water to thereby form a reaction mixture including a radiolabeled 3'-deoxy-3'-[¹⁸F]Fluorothymidine, [¹⁸F] ions, and residual reaction impurities;
passing the reaction mixture through a solid phase extraction cartridge as a first extraction step, the solid phase extraction cartridge including a solid sorbent to adsorb the radiolabeled 3'-deoxy-3'-[¹⁸F]Fluorothymidine on the sorbent, at least a portion of the residual reaction impurities passing through solid phase extraction cartridge without adsorbing on the sorbent;
washing off any remaining impurities on the sorbent with wash water;
eluting the radiolabeled 3'-deoxy-3'-[¹⁸F]Fluorothymidine off the sorbent using an alcohol solvent; and
drying and reconstituting in water the eluted radiolabeled 3'-deoxy-3'-[¹⁸F]Fluorothymidine before any further extraction steps,
wherein the method further comprises:
passing the reconstituted radiolabeled 3'-deoxy-3'-[¹⁸F]Fluorothymidine through a solid phase extraction cartridge; and further wherein high pressure liquid chromatography is not used in the method.

2. The method of claim 1 wherein:
the solid phase extraction cartridge sorbent is a reversed-phase sorbent.

3. The method of claim 1 wherein:
the alcohol solvent is an alkanol.

4. The method of claim 1 wherein:
the reaction solvent is an aprotic solvent.

5. The method of claim 1 wherein:
the reaction solvent is dimethyl sulfoxide.

6. The method of claim 5 wherein:
the dilution water is in an amount in excess of the reaction solvent.

7. The method of claim 6 wherein:
the volume ratio of the dilution water to the reaction solvent is at least 7 to 1.

8. The method of claim 1 wherein:
the wash water is not delivered by way of the reaction vessel.

9. The method of claim 1 further comprising:
passing the reaction mixture through a filter before passing the reaction mixture through the solid phase extraction cartridge, the filter removing precipitates from the reaction mixture.

10. The method of claim 9 further comprising:
bypassing the filter after passing the reaction mixture through the filter for a time period.

11. The method of claim 1 wherein:
the steps of passing the reaction mixture through the solid phase extraction cartridge, washing off the remaining impurities on the sorbent, and eluting the radiolabeled 3'-deoxy-3'-[¹⁸]Fluorothymidine off the sorbent take two minutes or less.

12. The method of claim 1 wherein:
wherein the solid phase extraction cartridge sorbent is a C-18 sorbent.

13. An apparatus for preparing 3'-deoxy-3'-[¹⁸F]Fluorothymidine, the apparatus comprising:
a reaction vessel for containing a fluorothymidine precursor, a reaction solvent for the fluorothymidine precursor, a source of [¹⁸F] ions, and dilution water to thereby form a reaction mixture including a radiolabeled 3'-deoxy-3'-[¹⁸F]Fluorothymidine, [¹⁸F] ions, and residual reaction impurities;
a solid phase extraction cartridge in fluid communication with the reaction vessel, the solid phase extraction cartridge including a solid sorbent to adsorb the radiolabeled 3'-deoxy-3'-[¹⁸F]Fluorothymidine on the sorbent wherein at least a portion of the residual reaction impurities pass through solid phase extraction cartridge without adsorbing on the sorbent;
a source of water in fluid communication with the solid phase extraction cartridge for providing water to wash off any remaining impurities on the sorbent with water; and
a source of an alcohol solvent in fluid communication with the solid phase extraction cartridge for providing the alcohol solvent to elute the radiolabeled 3'-deoxy-3'-[¹⁸F]Fluorothymidine off the sorbent using the alcohol solvent,
wherein the apparatus does not include a high pressure liquid chromatography unit.

14. The method according to claim 1 for preparing 3'-deoxy-3'-[¹⁸F]Fluorothymidine on a radiolabeled product synthesizer including a reaction vessel and a high pressure liquid chromatography unit in a product flow path, the method comprising:
removing the high pressure liquid chromatography unit from the product flow path;
combining in the reaction vessel a fluorothymidine precursor, a reaction solvent for the fluorothymidine precursor, a source of [¹⁸F] ions, and dilution water to thereby form a reaction mixture including a radiolabeled 3'-deoxy-3'-[¹⁸F]Fluorothymidine, [¹⁸F] ions, and residual reaction impurities;
passing the reaction mixture through a solid phase extraction cartridge, the solid phase extraction cartridge including a solid sorbent to adsorb the radiolabeled 3'-deoxy-3'-[¹⁸F]Fluorothymidine on the sorbent, at least a portion of the residual reaction impurities passing through solid phase extraction cartridge without adsorbing on the sorbent;
washing off any remaining impurities on the sorbent with wash water; and
eluting the radiolabeled 3'-deoxy-3'-[¹⁸F]Fluorothymidine off the sorbent using an alcohol solvent.

15. The method of claim 14 wherein:
the alcohol solvent is ethanol.

16. The method of claim 14 wherein:
the reaction solvent is dimethyl sulfoxide.

17. The method of claim 16 wherein:
the dilution water is in an amount in excess of the reaction solvent.

18. The method of claim 14 wherein:
the wash water is not delivered by way of the reaction vessel.

19. The method of claim 14 further comprising:
passing the reaction mixture through a filter before passing the reaction mixture through the solid phase extraction cartridge, the filter removing precipitates from the reaction mixture.

20. The method of claim 19 further comprising:
bypassing the filter after passing the reaction mixture through the filter for a time period.

21. The method of claim 14 wherein:
the steps of passing the reaction mixture through a solid phase extraction cartridge, washing off the remaining impurities on the sorbent, and eluting the radiolabeled 3'-deoxy-3'-[¹⁸F]Fluorothymidine off the sorbent take two minutes or less.

22. The method of claim 14 wherein:
wherein the solid phase extraction cartridge sorbent is a C-18 sorbent.

## Patentansprüche

1. Verfahren zum Zubereiten von 3'-Deoxy-3'-[¹⁸F]-Fluorthymidin, wobei das Verfahren umfasst:
Kombinieren eines Fluorthymidin-Precursors, eines Reaktionslösungsmittels für den Fluorthymidin-Precursor, einer Quelle für [¹⁸F]-lonen und von Verdünnungswasser, um dadurch ein Reaktionsgemisch zu bilden, das radioaktiv markiertes 3'-Deoxy-3'-[¹⁸F]-Fluorthymidin, [¹⁸F]-lonen und restliche Reaktionsverunreinigungen enthält;
Leiten des Reaktionsgemischs durch eine Festphasen-Extraktionskartusche als einen ersten Extraktionsschritt, wobei die Festphasen-Extraktionskartusche ein festes Sorbens enthält, um das radioaktiv markierte 3'-Deoxy-3'-[¹⁸F]-Fluorthymidin an dem Sorbens zu adsorbieren, wobei wenigstens ein Teil der restlichen Reaktionsverunreinigungen ohne Adsorption an dem Sorbens durch die Festphasen-Extraktionskartusche gehen;
Abspülen irgendwelcher verbleibenden Verunreinigungen an dem Sorbens mit Spülwasser;
Eluieren des radioaktiv markierten 3'-Deoxy-3'-[¹⁸F]-Fluorthymidins aus dem Sorbens unter Verwendung eines Alkohollösungsmittels; und
Trocknen und Wiederherstellen des eluierten radioaktiv markierten 3'-Deoxy-3'-[¹⁸F]-Fluorthymidins in Wasser vor irgendwelchen weiteren Extraktionsschritten,
wobei das Verfahren ferner umfasst:
Leiten des wiederhergestellten radioaktiv markierten 3'-Deoxy-3'-[¹⁸F]-Fluor-thymidins durch eine Festphasen-Extraktionskartusche; und wobei in dem Verfahren ferner keine Hochdruckflüssigkeitschromatographie verwendet wird.

2. Verfahren gemäß Anspruch 1, wobei:
das Festphasen-Extraktionskartuschen-Sorbens ein Sorbens mit umgekehrten Phasen ist.

3. Verfahren gemäß Anspruch 1, wobei:
das Alkohollösungsmittel ein Alkohol ist.

4. Verfahren gemäß Anspruch 1, wobei:
das Reaktionslösungsmittel eine protonenfreies Lösungsmittel ist.

5. Verfahren gemäß Anspruch 1, wobei:
das Reaktionslösungsmittel Dimethylsulfoxid ist.

6. Verfahren gemäß Anspruch 5, wobei:
das Verdünnungswasser eine größere Menge als die des Reaktionslösungsmittels ist.

7. Verfahren gemäß Anspruch 6, wobei:
das Volumenverhältnis des Verdünnungswassers zu dem Reaktionslösungsmittel wenigstens 7 zu 1 ist.

8. Verfahren gemäß Anspruch 1, wobei:
das Spülwasser nicht über das Reaktionsgefäß geliefert wird.

9. Verfahren gemäß Anspruch 1, das ferner umfasst:
Leiten des Reaktionsgemischs durch einen Filter, bevor das Reaktionsgemisch durch die Festphasen-Extraktionskartusche geht, wobei der Filter Niederschläge aus dem Reaktionsgemisch entfernt.

10. Verfahren gemäß Anspruch 9, das ferner umfasst:
Umgehen des Filters, nachdem das Reaktionsgemisch für eine Zeitdauer durch den Filter gegangen ist.

11. Verfahren gemäß Anspruch 1, wobei:
die Schritte des Leitens des Reaktionsgemischs durch die Festphasen-Extraktionskartusche, des Abspülens der verbleibenden Verunreinigungen an dem Sorbens und des Eluierens des radioaktiv markierten 3'-Deoxy-3'-[¹⁸F]-Fluorthymidins aus dem Sorbens zwei Minuten oder weniger dauern.

12. Verfahren gemäß Anspruch 1, wobei:
das Festphasen-Extraktionskartuschen-Sorbens ein C-18-Sorbens ist.

13. Vorrichtung zum Zubereiten von 3'-Deoxy-3'-[¹⁸F]-Fluorthymidin, wobei die Vorrichtung umfasst:
ein Reaktionsgefäß zum Aufnehmen eines Fluorthymidin-Precursors, eines Reaktionslösungsmittels für den Fluorthymidin-Precursor, einer Quelle für [¹⁸F]-Ionen und von Verdünnungswasser, um dadurch ein Reaktionsgemisch zu bilden, das ein radioaktiv markiertes 3'-Deoxy-3'-[¹⁸F]-Fluorthymidin, [¹⁸F]-Ionen und restliche Reaktionsverunreinigungen enthält;
eine Festphasen-Extraktionskartusche in Fluidverbindung mit dem Reaktionsgefäß, wobei die Festphasen-Extraktionskartusche ein festes Sorbens enthält, um das radioaktiv markierte 3'-Deoxy-3'-[¹⁸F]-Fluorthymidins an dem Sorbens zu adsorbieren, wobei wenigstens ein Teil der restlichen Reaktionsverunreinigungen ohne Adsorption an dem Sorbens durch die Festphasen-Extraktionskartusche gehen;
eine Quelle für Wasser in Fluidverbindung mit der Festphasen-Extraktionskartusche zum Bereitstellen von Wasser zum Abspülen irgendwelcher verbleibender Verunreinigungen an dem Sorbens mit Wasser; und
eine Quelle für ein Alkohollösungsmittel in Fluidverbindung mit der Festphasen-Extraktionskartusche zum Bereitstellen des Alkohollösungsmittels zum Eluieren des radioaktiv markierten 3'-Deoxy-3'-[¹⁸F]-Fluorthymidins aus dem Sorbens unter Verwendung des Alkohollösungsmittels,
wobei die Vorrichtung keine Hochdruckflüssigkeitschromatographie-Einheit enthält.

14. Verfahren gemäß Anspruch 1 zum Zubereiten von 3'-Deoxy-3'-[¹⁸F]-Fluorthymidin in einer Syntheseeinrichtung für radioaktiv markierte Produkte, die ein Reaktionsgefäß und eine Hochdruckflüssigkeitschromatographie-Einheit in einem Produktflussweg enthält, wobei das Verfahren umfasst:
Entfernen der Hochdruckflüssigkeitschromatographie-Einheit aus dem Produktflussweg;
Kombinieren eines Fluorthymidin-Precursors, eines Reaktionslösungsmittels für den Fluorthymidin-Precursor, einer Quelle für [¹⁸F]-lonen und von Verdünnungswasser in dem Reaktionsgefäß, um dadurch ein Reaktionsgemisch zu bilden, das radioaktiv markiertes 3'-Deoxy-3'-[¹⁸F]-Fluorthymidin, [¹⁸F]-lonen und restliche Reaktionsverunreinigungen enthält;
Leiten des Reaktionsgemischs durch eine Festphasen-Extraktionskartusche, wobei die Festphasen-Extraktionskartusche ein festes Sorbens enthält, um das radioaktiv markierte 3'-Deoxy-3'-[¹⁸F]-Fluorthymidins an dem Sorbens zu adsorbieren, wobei wenigstens ein Teil der restlichen Reaktionsverunreinigungen ohne Absorption an dem Sorbens durch die Festphasen-Extraktionskartusche gehen;
Abspülen irgendwelcher verbleibender Verunreinigungen an dem Sorbens mit Spülwasser; und
Eluieren des radioaktiv markierten 3'-Deoxy-3'-[¹⁸F]-Fluorthymidins aus dem Sorbens unter Verwendung eines Alkohollösungsmittels.

15. Verfahren gemäß Anspruch 14, wobei:
das Alkohollösungsmittel Ethanol ist.

16. Verfahren gemäß Anspruch 14, wobei:
das Reaktionslösungsmittel Dimethylsulfoxid ist.

17. Verfahren gemäß Anspruch 16, wobei:
das Verdünnungswasser eine größere Menge als die des Reaktionslösungsmittels ist.

18. Verfahren gemäß Anspruch 14, wobei:
das Spülwasser nicht über das Reaktionsgefäß geliefert wird.

19. Verfahren gemäß Anspruch 14, das ferner umfasst:
Leiten des Reaktionsgemischs durch einen Filter, bevor das Reaktionsgemisch durch die Festphasen-Extraktionskartusche geht, wobei der Filter Niederschläge aus dem Reaktionsgemisch entfernt.

20. Verfahren gemäß Anspruch 19, das ferner umfasst:
Umgehen des Filters, nachdem das Reaktionsgemisch für eine Zeitdauer durch den Filter gegangen ist.

21. Verfahren gemäß Anspruch 14, wobei:
die Schritte des Leitens des Reaktionsgemischs durch eine Festphasen-Extraktionskartusche, des Abspülens der verbleibenden Verunreinigungen an dem Sorbens und des Eluierens des radioaktiv markierten 3'-Deoxy-3'-[¹⁸F]-Fluorthymidins aus dem Sorbens zwei Minuten oder weniger dauern.

22. Verfahren gemäß Anspruch 14, wobei:
das Festphasen-Extraktionskartuschen-Sorbens ein C-18-Sorbens ist.

## Revendications

1. Procédé de préparation de 3'-désoxy-3'-[¹⁸F]fluorothymidine, le procédé comprenant les étapes suivantes :
combinaison d'un précurseur de la fluorothymidine, d'un solvant de réaction pour le précurseur de la fluorothymidine, d'une source d'ions [¹⁸F] et une eau de dilution pour ainsi former un mélange réactionnel incluant une 3'-désoxy-3'-[¹⁸F]fluorothymidine radiomarquée, des ions [¹⁸F] et des impuretés de réaction résiduelles ;
passage du mélange réactionnel dans une cartouche d'extraction en phase solide comme première étape d'extraction, la cartouche d'extraction en phase solide incluant un sorbant solide pour adsorber la 3'-désoxy-3'-[¹⁸F]fluorothymidine radiomarquée sur le sorbant, une partie au moins des impuretés de réaction résiduelles passant dans la cartouche d'extraction en phase solide sans être adsorbées sur le sorbant ;
élimination de toute impureté restante sur le sorbant par lavage avec une eau de lavage ;
élution de la 3'-désoxy-3'-[¹⁸F]fluorothymidine radiomarquée du sorbant en utilisant un solvant alcoolique ; et
séchage et reconstitution dans l'eau de la 3'-désoxy-3'-[¹⁸F]fluorothymidine radiomarquée éluée avant toute étape d'extraction supplémentaire,
où ledit procédé comprend en outre :
le passage de la 3'-désoxy-3'-[¹⁸F]fluorothymidine radiomarquée reconstituée dans une cartouche d'extraction en phase solide ; et où le procédé n'utilise pas la chromatographie liquide haute pression.

2. Procédé de la revendication 1, où :
le sorbant de la cartouche d'extraction en phase solide est un sorbant à phase inverse.

3. Procédé de la revendication 1, où :
le solvant alcoolique est un alcanol.

4. Procédé de la revendication 1, où :
le solvant de réaction est un solvant aprotique.

5. Procédé de la revendication 1, où :
le solvant de réaction est le sulfoxyde diméthylique.

6. Procédé de la revendication 5, où :
l'eau de dilution est présente à une quantité en excès de celle du solvant de réaction.

7. Procédé de la revendication 6, où :
le rapport en volumes entre l'eau de dilution et le solvant de réaction est d'au moins 7/1.

8. Procédé de la revendication 1, où :
l'apport en eau de lavage ne se fait pas par le biais de la cuve de réaction.

9. Procédé de la revendication 1, qui comprend en outre :
le passage du mélange réactionnel dans un filtre avant le passage du mélange réactionnel dans la cartouche d'extraction en phase solide, le filtre éliminant les précipités du mélange réactionnel.

10. Procédé de la revendication 9, qui comprend en outre :
le contournement du filtre après passage du mélange réactionnel dans le filtre pendant une certaine période de temps.

11. Procédé de la revendication 1, où :
les étapes de passage du mélange réactionnel dans la cartouche d'extraction en phase solide, d'élimination des impuretés restantes sur le sorbant par lavage et d'élution de la 3'-désoxy-3'-[¹⁸F]fluorothymidine radiomarquée du sorbant prennent deux minutes ou moins.

12. Procédé de la revendication 1, où :
le sorbant de la cartouche d'extraction en phase solide est un sorbant C-18.

13. Appareillage pour la préparation de 3'-désoxy-3'-[¹⁸F]fluorothymidine, l'appareillage comprenant :
une cuve de réaction destinée à contenir un précurseur de la fluorothymidine, un solvant de réaction pour le précurseur de la fluorothymidine, une source d'ions [¹⁸F] et une eau de dilution pour ainsi former un mélange réactionnel incluant une 3'-désoxy-3'-[¹⁸F]fluorothymidine radiomarquée, des ions [¹⁸F] et des impuretés de réaction résiduelles ;
une cartouche d'extraction en phase solide en communication fluidique avec la cuve de réaction, la cartouche d'extraction en phase solide incluant un sorbant solide pour adsorber la 3'-désoxy-3'-[¹⁸F]fluorothymidine radiomarquée sur le sorbant, ou une partie au moins des impuretés de réaction résiduelles passent dans la cartouche d'extraction en phase solide sans être adsorbées sur le sorbant ;
une source d'eau en communication fluidique avec la cartouche d'extraction en phase solide pour fournir l'eau servant à l'élimination de toute impureté restante sur le sorbant par lavage à l'eau ; et
une source d'un solvant alcoolique en communication fluidique avec la cartouche d'extraction en phase solide pour fournir le solvant alcoolique servant à l'élution de la 3'-désoxy-3'-[¹⁸F]fluorothymidine radiomarquée du sorbant en utilisant le solvant alcoolique,
où l'appareillage n'inclut pas une unité de chromatographie liquide haute pression.

14. Procédé selon la revendication 1 pour la préparation de 3'-désoxy-3'-[¹⁸F]fluorothymidine sur un dispositif de synthèse d'un produit radiomarqué qui inclut une cuve de réaction et une unité de chromatographie liquide haute pression dans un trajet d'écoulement du produit, le procédé comprenant les étapes suivantes :
retrait de l'unité de chromatographie liquide haute pression du trajet d'écoulement du produit ;
combinaison, dans la cuve de réaction, d'un précurseur de la fluorothymidine, d'un solvant de réaction pour le précurseur de la fluorothymidine, d'une source d'ions [¹⁸F] et d'une eau de dilution pour ainsi former un mélange réactionnel incluant une 3'-désoxy-3'-[¹⁸F]fluorothymidine radiomarquée, des ions [¹⁸F] et des impuretés de réaction résiduelles ;
passage du mélange réactionnel dans une cartouche d'extraction en phase solide, la cartouche d'extraction en phase solide incluant un sorbant solide pour adsorber la 3'-désoxy-3'-[¹⁸F]fluorothymidine radiomarquée sur le sorbant, une partie au moins des impuretés de réaction résiduelles passant dans la cartouche d'extraction en phase solide sans être adsorbées sur le sorbant ;
élimination de toute impureté restante sur le sorbant par lavage avec une eau de lavage ; et
élution de la 3'-désoxy-3'-[¹⁸F]fluorothymidine radiomarquée du sorbant en utilisant un solvant alcoolique.

15. Procédé de la revendication 14, où :
le solvant alcoolique est l'éthanol.

16. Procédé de la revendication 14, où :
le solvant de réaction est le sulfoxyde diméthylique.

17. Procédé de la revendication 16, où :
l'eau de dilution est présente à une quantité en excès de celle du solvant de réaction.

18. Procédé de la revendication 14, où :
l'apport en eau de lavage ne se fait pas par le biais de la cuve de réaction.

19. Procédé de la revendication 14, qui comprend en outre :
le passage du mélange réactionnel dans un filtre avant le passage du mélange réactionnel dans la cartouche d'extraction en phase solide, le filtre éliminant les précipités du mélange réactionnel.

20. Procédé de la revendication 19, qui comprend en outre :
le contournement du filtre après passage du mélange réactionnel dans le filtre pendant une certaine période de temps.

21. Procédé de la revendication 14, où :
les étapes de passage du mélange réactionnel dans la cartouche d'extraction en phase solide, d'élimination des impuretés restantes sur le sorbant par lavage et d'élution de la 3'-désoxy-3'-[¹⁸F]fluorothymidine radiomarquée du sorbant prennent deux minutes ou moins.

22. Procédé de la revendication 14, où :
le sorbant de la cartouche d'extraction en phase solide est un sorbant C.
